# EUROPEAN PATENT SPECIFICATION

(11) **EP 1 492 801 B1**
(45) Date of publication and mention of the grant of the patent: **07.01.2009**
(21) Application number: 03714505.9
(22) Date of filing: 04.04.2003
(51) Int. Cl.: C07H 5/02, A23L 1/236, A23L 2/60, A21D 2/12

(54) **METHODS AND COMPOSITIONS FOR ALTERING THE SWEETNESS DELIVERY PROFILE OF SUCRALOSE**
VERFAHREN UND ZUSAMMENSETZUNG ZUR VERÄNDERUNG DES SÜSSGESCHMACK-PROFILS VON SUCRALOSE
PROCEDES ET COMPOSITIONS PERMETTANT D'ALTERER LE PROFIL DE L'EFFET EDULCORANT DE LA SUCRALOSE

(30) Priority: 05.04.2002 US 116759
(43) Date of publication of application: 05.01.2005
(73) Proprietor: Tate & Lyle Technology Limited, London EC3R 6DQ (GB)
(72) Inventor: MERKEL, Carolyn, M., North Haledon, NJ 07508 (US); CATANI, Steven, J., Athens, GA 30606 (US); FRY, John, C., Horsham, West Sussex RH123HQ (GB)
(74) Representative: Smyth, Gyles Darren
(86) International application number: PCT/US2003/010210
(87) International publication number: WO 2003/087116

(56) References cited:
- EP-A- 0 043 649
- EP-A- 0 064 361
- EP-A- 0 354 680
- EP-A- 0 457 724
- WO-A-00/01253
- US-A- 4 405 654
- US-A- 5 298 611

## Description

### FIELD OF THE INVENTION

The present invention teaches novel compositions and methods for altering the sweetness delivery profile of 4, 1', 6' -trichloro-4, 1', 6'-trideoxygalactosucrose. This invention also teaches novel uses for compositions comprising 4, 1', 6'-trichloro-4, 1', 6'-trideoxygalactosucrose.

### BACKGROUND OF THE INVENTION

Sucralose, a sweetener with a sweetness intensity several hundred times that of sucrose, is derived from sucrose by replacing the hydroxyl groups in its 4, 1', and 6' positions with chlorine. Synthesis of sucralose is technically challenging because of the need to selectively replace specific hydroxyl groups with chlorine atoms, while preserving other hydroxyl groups including a highly reactive primary hydroxyl group. Numerous approaches to this synthesis have been developed. *See, e.g.,* U.S. Pat. Nos. 4,343,934; 4,362,869; 4,826,962; 4,980,463; and 5,141,860. Such approaches typically provide a product that contains varying levels of other halogenated sugar derivatives in addition to sucralose. The types of halogenated sugar compounds present in product mixture may vary according to the synthetic and purification routes used and the particular conditions of the synthesis and purification processes.

Combinations of sweeteners and other substances are known to those of ordinary skill in the art. WO 00/01253, which is expressly incorporated by reference herein, relates to methods for altering the sweetness delivery profile of sucralose via the combination of sucralose and 2,4-dihydroxybenzoic acid. Similarly, WO 93/10677, which is expressly incorporated by reference herein, relates to a wide range of indigestible compounds that are effective eliminators of undesirable components of the sweetness delivery profile of sucralose.

Scant attention has been paid, however, to combinations of 4, 1', 6'-trichloro-4, 1', 6'-trideoxygalactosucrose and other halogenated sugar derivatives. The present inventions provide mixtures of 4, 1', 6'-trichloro-4, 1', 6'-trideoxygalactosucrose and halogenated sugar derivatives as well as methods of altering the sweetness delivery profile of 4, 1', 6'-trichloro-4, 1' ,6'-trideoxygalactosucrose via its combination with other halogenated sugar derivatives.

### SUMMARY OF THE INVENTION

The present invention relates to a composition comprising 4, 1', 6'-trichloro-4, 1', 6'-trideoxygalactosucrose in an amount from 98.0% to 99.999% of said composition by weight, at least one halogenated sugar derivative of the following formula (I), and at least one halogenated sugar derivative selected from the group of the following formulae (II-III) : 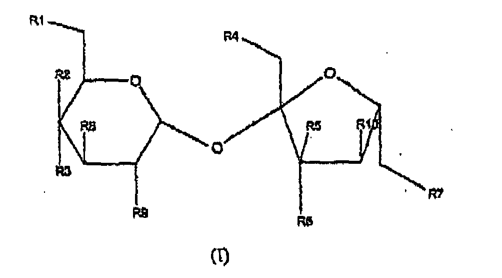 wherein
R1 represents a hydroxy group, an acyl group, or a halogen atom;
R2 and R3 respectively represent:
   (a) a hydrogen atom and a hydroxy group;
   (b) a hydrogen and a halogen atom ;
   (c) a halogen atom and a hydrogen atom; or
   (d) a hydroxy group and a hydrogen atom;
R4, R5, R6, R7, R8, R9 and R10 represent a hydrogen atom, a hydroxy group, or a halogen atom; and 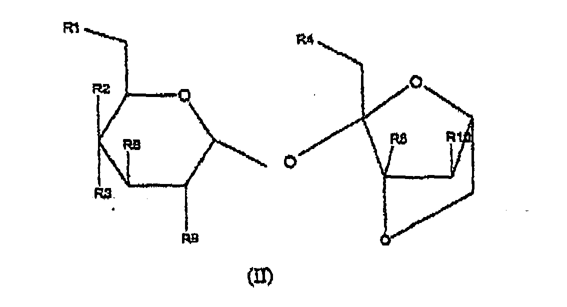
wherein
R1 represents a hydroxy group, an acyl group, or a halogen atom;
R2 and R3 respectively represent:
   (a) a hydrogen atom and a hydroxy group;
   (b) a hydrogen and a halogen atom;
   (c) a halogen atom and a hydrogen atom; or
   (d) a hydroxy group and a hydrogen atom;
R4, R5, R8, R9 and R10 represent a hydrogen atom, a hydroxy group, or a halogen atom; and 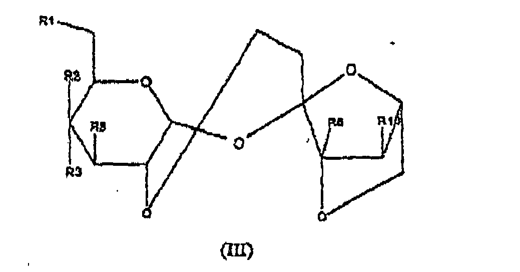
wherein
R1 represents a hydroxy group, an acyl group, or a halogen atom;
R2 and R3 respectively represent:
   (a) a hydrogen atom and a hydroxy group;
   (b) a hydrogen and a halogen atom;
   (c) a halogen atom and a hydrogen atom; or
   (d) a hydroxy group and a hydrogen atom; and
R5, R8, and R10 represent a hydrogen atom, a hydroxy group, or a halogen atom.

In this embodiment, the halogenated sugar derivative may be in an amount from about 0.001 % to about 2.0% of the composition by weight and the 4, 1', 6'-trichloro-4,1', 6-trideoxygalactosucrose may be in an amount from about 98.0% to about 99. 999% of the composition by weight. In another embodiment, the weight ratio of the halogenated sugar derivative to the 4,1',6'-trichloro-4,1', 6'-trideoxygalactosucrose may be from about 1: 99,999 to about 1: 50.

Other embodiments of the present invention may include consumer products, combination sweeteners, and beverages comprising the compositions of the present invention.

The present invention also relates to any composition comprising 4, 1', 6'-trichloror-4,1',6'-trideoxygalactosucrose and at least one halogenated sugar derivative wherein the halogenated sugar derivative may be in an amount from about 0.001% to about 2.0% of the composition by weight and wherein the 4, 1', 6'-trichloro-4, 1', 6'-trideoxygalactosucrose may be in an amount from about 98.0% to about 99.999% of the composition by weight.

In addition, the present invention relates to any composition comprising 4, 1', 6'-trichloro-4, 1', 6'-trideoxygalactosucrose and at least one halogenated sugar derivative wherein the weight ratio of the halogenated sugar derivative to the 4, 1', 6'-trichloro-4, 1', 6'-trideoxygalactosucrose is from about 1:99,999 to about 1:50. Other embodiments of the present invention may include consumer products, combination sweeteners, and beverages comprising the compositions of the present invention.

Yet another embodiment of the present invention comprises methods for altering the sweetness delivery profile of 4, 1', 6'-trichloro-4, 1', 6'-trideoxygalactosucrose comprising the step of supplying a composition comprising 4, 1', 6'-trichloro-4, 1', 6'-trideoxyganlactosucrose and at least one halogenated sugar derivative wherein the weight ratio of the halogenated sugar derivative to the 4, 1', 6'-trichloro-4, 1', 6'-trideoxygalactosucrose is from about 1:99,999 to about 1:50.

### DETAILED DESCRIPTION OF THE INVENTION

It is understood that the present invention is not limited to the particular methodologies, protocols, pH, and reagents, etc., described herein, as these may vary. It is also to be understood that the terminology used herein is used for the purpose of describing particular embodiments only, and is not intended to limit the scope of the present invention. As used herein and in the appended claims, the singular forms "a," "an," and "the" include plural reference unless the context clearly dictates otherwise. Thus, for example, a reference to "a halogenated sugar derivative^{a} is a reference to one or more halogenated sugar derivatives and includes equivalents thereof known to those skilled in the art and so forth.

Unless defined otherwise, all technical and scientific terms used herein have the same meanings as commonly understood by one of ordinary skill in the art to which this invention belongs. Numerous methods, devices, and materials are described herein, although any methods and materials similar or equivalent to those described herein can be used in the practice or testing of the present invention. All documents cited herein are incorporated by reference herein in their entirety.

### Definitions

**4, 1', 6'-trichloro-4, 1', 6'-trideoxygalackosucrose:** as used herein, refers to a preparation of sucralose in which other compounds such as, for example, one or more halogenated sugar derivatives, do not exist.

**"Alter," "altering":** as used herein suggests any action to make different or change. As used herein, "altering the sweetness delivery profile of sucralose," may include activities that, for example, change the sweetness delivery profile of sucralose, such as activities that affect characteristics of sucralose such as sweetness acceptability, sweetness onset, sweetness intensity, aftertaste intensity, bitterness, sweetness duration, pleasantness of aftertaste, overall liking, and quality of taste as "artificial" or "natural."

**Beverage:** as used herein includes any non-carbonated or carbonated beverage such as cola, diet cola, soda, diet soda, juice cocktail, root beer, birch beer, any fountain drink, sparkling fruit juice, water, sparkling water, tonic water, sport drink, fruit juices, isotonic beverages and club soda. Beverage may also include any fermented or non-fermented drink such as any beer, including ale, pilsner, lager, or derivation thereof, malt liquor, red wine, white wine, sparkling wine, fortified wine, wine cooler, wine spritzer, any pre-made cocktail mixer including margarita mix, sour mix, or daiquiri mix, any fermented fruit or tea beverage, hard liquor, and any flavored liqueur such as brandy, schnapps, bitters, or cordial. Beverage may include any liquid or dry dairy, milk, or cream product or any liquid or dry dairy, cream, or milk substitute such as half & half, non-dairy creamer, powdered creamer, flavored creamer, soy milk product, and lactose-reduced milk product and the like. Beverage may also include any fruit or vegetable juice in whole, concentrated, or powdered form and any combination of fruit and vegetable juices or other beverages. Beverage may also include coffee, any coffee drink, any coffee flavoring syrup, tea, iced tea, and cocoa, as well as any combination of any of the foregoing in powdered or liquid form. Beverage may also include powdered drink mixes of any flavors, including mixes requiring the addition of a sweetener before or after reconstitution to fluid form.

**Combination sweetener:** as used herein includes any combination or permutation of sweeteners, including combinations of sucralose, saccharin, aspartame, acesulfame potassium, cyclamate, alitame, neotame, stevioside, glucose, fructose, levulose, maltose, lactose, any sugar alcohol, sorbitol, xylitol, and mannitol. Combination sweeteners may be granular in form, but may be in any other suitable form such as powder, liquid or syrup. The combination sweetener may consist essentially of sucralose. The combination sweetener may consist essentially of sucralose and a carrier such as dextrose, lactose, maltodextrin or water.

**Consumer product:** as used herein includes fruit products such as applesauce, jams, jellies, marmalades, fruit snacks, fruit butters, and fruit spreads. Consumer product may also include any viscous or solid dairy, milk, or cream product, such as cheese, ice cream, ice milk, frozen yogurt, yogurt, and the like. Consumer product also includes baked goods such as breads, doughnuts, cakes, cheesecakes, tianisines, pasitries, pies, bageis, cookies, scones, crackers, muffins, and wafers. Consumer product includes cereal products such as ready-to-eat cold cereals, grits, hot cereals, granola mixes, oatmeal, and trail mixes. Consumer product includes condiments such as butter, peanut butter, whipped cream, dulce de leche, sour cream, BBQ sauce, chill, syrup, gravy, mayonnaise, olives, seasonings, relish, pickles, sauces, snack dips, ketchup, salsa, mustard, salad dressings, and pickled peppers. Consumer product includes snack foods and confectionary products such as apple bars, pudding, candy bars, hard candy, chocolate products, lollipops, fruit chews, marshmallows, chewing gum, bubble gum, gummy bears, jelly beans, caramel, taffy, pie fillings, syrups, gel snacks, mints, popcorn, chips, and pretzels. Consumer product includes meat products such as hot dogs, canned fish, sausage, prepared meats, canned meat, dehydrated meat, and luncheon meat. Consumer product includes soups, consommé, and bouillon. Consumer product includes dental products such as toothpaste, dental floss, mouthwash, denture adhesive, enamel whitener, fluoride treatments, and oral care gels. Consumer product includes cosmetic and beauty aids such as lipstick, lip balm, lip gloss, and petroleum jelly. Consumer product includes therapeutic items such as non-tobacco snuff, tobacco substitutes, pharmaceutical compositions, chewable medications, cough syrups, throat sprays, throat lozenges, cough drops, antibacterial products, pill coatings, gel caplets, soluble fiber preparations, antacids, tablet cores, rapidly absorbed liquid compositions, stable foam compositions, rapidly disintegrating pharmaceutical dosage forms, beverage concentrates for medicinal purposes, aqueous pharmaceutical suspensions, liquid concentrate compositions, and stabilized sorbic acid solutions, phosphate buffers, saline solutions, emulsions, non-aqueous pharmaceutical solvents (propylene glycol, polyethylene glycol, vegetable oils), aqueous pharmaceutical carriers (water, alcohol), and solid pharmaceutical carriers (lactose, cellulose), and pharmaceutical preservatives/additives (antimicrobials, antioxidants, chelating agents, inert gases, flavoring agents, coloring agents). Consumer product includes nutritional products such as meal replacement bars, meal replacement shakes, dietary supplements, protein mixes, protein bars, carbohydrate control bars, low carbohydrate bars, meal supplements, electrolyte solutions, whey protein products, metabolic response modifiers, appetite control beverages, and echinacea sprays. Consumer product includes animal foodstuffs such as dog and cat food, rat feed, cattle feed, pig feed, and bird feed. Consumer product includes foodstuffs such as baby food, infant formulae, and other products for infant health and nutrition, such as oral rehydration beverages. Consumer product includes tobacco products such as pipe tobacco, cigarette tobacco, and chewing tobacco. Consumer product includes any substance intended for oral consumption either alone or with another substance. Consumer product includes any composition intended for oral, parenteral, intravenous, subcutaneous, intramuscular, intraorbital, intraspinal, intrasternal, or intruarterial administration to a human or other animal such as livestock or a domestic animal. A consumer product may optionally include additional agents such as carriers (starch, lactose, and sucrose), bulking agents (maltodextrins), adjuvants (Indocyanine green, vanilla, and oil of wintergreen), coloring agents, viscosity-adjusting agents including soluble cellulose derivatives (carboxy-methylcellulose), thickening gums (xanthan, gellan, carrageenan), and synthetic food additive materials (polyoxyethylene, carbomer).

**Halogenated sugar derivative:** as used herein includes any derivative of a sugar in which one or more hydroxyl groups within the sugar have been replaced with a halogen such as chlorine, bromine, iodine, or fluorine. Halogenated sugar derivative includes any derivative of sucrose in which one or more hydroxyl groups within sucrose have been replaced with a halogen such as chlorine, bromine, iodine, or fluorine. Halogenated sugar derivative includes any derivative of galactosucrose in which one or more hydroxyl groups within galactosucrose have been replaced with a halogen such as chlorine, bromine, iodine, or fluorine. Halogenated sugar derivative does not include sucralose.

**SPLENDA**®**:** as used herein refers to a commercial brand of sucralose available from Splenda, Inc. (Fort Washington, Pa. 19034). SPLENDA® Brand Sweetener (sucralose) is supplied either as a crystalline powder or as a 25% liquid concentrate solution of sucralose.

**Sucralose:** as used herein, refers to a preparation of 4, 1', 6'-trichloro-4, 1', 6'-trideoxygalactosucrose in which other compounds such as, for example, one or more halogenated sugar derivatives, might exist.

**Sweetness delivery profile:** as used herein includes both the time period preceding sweetness onset ("onset period") and the time period during which sweetness lingers ("lingering period"). Reducing or lengthening either period alters the sweetness delivery profile of a sucralose-containing composition. Thus, in one embodiment of the invention, the alteration comprises reducing the onset period. In another embodiment, the alteration comprises reducing the lingering period. In one embodiment, the alteration comprises reducing both the onset period and the lingering period. Sweetness delivery profile includes other characteristics of compounds such as sweetness acceptability, sweetness onset, sweetness intensity, aftertaste Intensity, bitterness, sweetness duration, pleasantness of aftertaste, overall liking, quality of taste as "artificial" or "natural," and others known to those skilled in the art.

The present invention teaches novel compositions comprising 4, 1', 6'-trichloro-4, 1', 6'-trideoxygalactosucrose and one or more halogenated sugar derivatives. The present invention also teaches methods for improving the sweetness delivery profile of 4, 1', 6'-trichloro-4, 1', 6'-trideoxygalactosucrose by combining it with one or more halogenated sugar derivatives.

High intensity sweeteners, such as sucralose, are reported to have sweetness delivery problems such as delayed onset and lingering of sweetness. Wiet et al., 58(3) J. Food Sci. 599-602 (1993). These phenomena arise via mechanisms which are biochemically distinct from those responsible for generating bitter or metallic aftertastes in response to certain other sweeteners. Lee, 45 Advances in Carbohydrate Chemistry and Biochemistry 199-351 (1987). It was discovered in this invention that halogenated sugar derivatives alter the sweetness delivery profile of 4, 1', 6'-trichloro-4, 1', 6'-trideoxygalactosucrose. Indeed, the sweetness delivery profile of 4, 1', 6'-trichloro-4, 1', 6'-trideoxygalactosucrose would not be expected to be altered by the presence of halogenated sugar derivatives from which 4, 1', 6'-trichloro-4, 1', 6'-trideoxygalacto-sucrose is ordinarily separated via extant purification processes. It was found that halogenated sugar derivatives affect one or more aspects of the sweetness delivery profile of 4, 1', 6'-trichloro-4, 1', 6'-trideoxygalactosucrose. Among these aspects are sweetness acceptability, sweetness onset, sweetness intensity, aftertaste intensity, bittemess, sweetness duration, pleasantness of aftertaste, overall liking, and quality of taste as "artificial" or "natural."

Accordingly, this invention provides methods of altering the sweetness delivery profile of 4, 1', 6'-trichloro-4, 1', 6'-trideoxygalactosucrose. Such methods comprise combining 4, 1', 6'-trichloro-4, 1', 6'-trideoxygalactosucrose with other halogenated sugar derivatives, specifically within a composition.

Sucralose, as well as other halogenated sugar derivatives, may be produced by a variety of techniques known in the art. A representative example of such techniques is found within U.S. Pat. No. 4,405,654, which is expressly incorporated by reference herein. This patent relates to processes for the synthesis of a variety of halogenated sugar derivatives. These derivatives include, for example, 4-bromo-1,6-dichloro-1,4,6-trideoxy- β-D-fructofuranosyl 4-chloro-4-deoxy-α-D-galactopyranoside; 1,4, 6-trideoxy-β-D-fructofuranosyl 4-bromo-4-deoxy-α-D-galactopyranoside; 4,1',4'-trichloro-4,1',4'-trideoxygalactosucrose; 4,4',6'-trichloro-4,4',- 6'-trideoxygalactosucrose; 1',4',6'-trichloro-1',4',6'-trideoxysucrose; 1,4,6-tribromo-1,4,6-trideoxy-β-D-fructofuranosyl 4-chloro-4-deoxy-α-D-galactopyranoside; 1,6-dichloro-1,4,6-trideoxy-4-iodo-β-D-fructofuranosyl 4-chloro-4-deoxy-α-D-galactopyranoside; 1',4'-dichloro-1',4'-dideoxysucrose; 1,4,6-trichloro-1,4,6-trideoxy-β-D-fructofuranosyl 4-deoxy-4-fluoro-α-D-galactopyranoside; and 4,1',4',6'-tetrachloro-4,1',4',6'-tetraceoxygalactosucrose.

U.S. Pat. No. 4,435,440, which is expressly incorporated by reference herein, also relates to processes for the synthesis of halogenated sugar derivatives. Such derivatives include, for example, 1'-chloro-1'-deoxysucrose, 4-chloro-4-deoxy-β-D-galactopyranosyl-β-D-fructofuranoside; 4-chloro-4-deoxy-α-D-galactopyranosyl-1-chloro-1-deoxy-β-D-fructofuranoside; 1',6'-dichloro-1',6'-dideoxysucrose; 4-chloro-4-deoxy-α-D-galactopyranosyl-1,6-dichloro-1,6-dideoxy-β-D-fructofuranoside; 4,6-dichloro-4,6-dideoxy-α-D-galactopyranosyl-6-chloro-6-deoxy-β-D-fructofuranoside; 6,1',6'-trichloro-6,1',6'-trideoxysucrose; 4,6-dichloro-4,6-dideoxy-α-D-galactopyranosyl-1,6-dichloro-1,6-dideoxy-β-D-fructofuranoside; and 4,6,1',6'-tetrachloro-4,6,1',6'-tetradeoxysucrose.

U.S. Pat. No. 4,980,463, which is expressly incorporated by reference herein, relates to a process in which sucralose and other halogenated sugar compounds are produced by KOH treatment of a methanol solution of sucralose-6-benzoate. The methanol is removed by evaporation, and the residue comprising halogenated sugar compounds is dissolved in water.

U.S. Pat. No. 5,034,551, which is expressly incorporated by reference herein, relates to a similar process in which a base is used to hydrolyze a solution of sucralose-6-benzoate in methanol. The methanol is removed by evaporation, and the halogenated sugar residues are dissolved in water.

U.S. Pat. No. 5,498,709, which is expressly incorporated by reference herein, relates to processes by which sucralose and other halogenated sugar derivatives are produced via the deacylation of 6-O-acyl-4,1',6'-trichloro-4,1',6'-trideoxygalactosucrose.

U.S. Pat. No. 5,530,106, which is expressly incorporated by reference herein, relates to the production of halogenated sugar derivatives by the Chlorination of sucrose-6-esters.

The U.S. patent application entitled "Extractive Methods for Purifying Sucralose," filed on Mar. 8, 2002, and U.S. Pat. No. 5,498,709, expressly incorporated by reference herein, relate to methods for the production of sucralose, as well as halogenated sugar derivatives such as, for example, dichlorosucrose acetate; 6,1',6'-trichlorosucrose, 4,6,6'-trichlorosucrose; 4,1',4',6'-tetrachlorogalactotagatose; 4,1',6'-trichlorogalactosucrose-6-acetate; 4,6,1',6'-tetrachlorogalactosucrose; 4,1'-dichlorogalactosucrose; 3',6'-dichloroanhydrosucrose; 4,6'-dichlorogalactosucrose; 1',6'-dichlorosucrose, 6,6'-dichlorosucrose; 4,1',6'-trichlorosucrose; 4,6,6'-trichlorogalactosucrose; 4,1',5'-trichlorogalactosucrose-6-acetate; and 4,6,6'-trichlorogalactosucrose.

The U.S. patent application entitled "Process for Improving Sucralose Purity and Yield," filed on Mar. 8, 2002, and U.S. Pat. No. 5,977,349, expressly incorporated by reference herein, relate to methods for the production of sucralose, as well as halogenated sugar derivatives such as, for example, 4,1'-dichlorogalactosucrose; 3',6'-anhydrogalactosucrose; 4,6'-dichlorogalactosucrose, 1',6'-dichlorosucrose, 6,6'-dichlorosucrose; 4,1',6'-trichlorosucrose; 6,1',6'-trichlorogalactosucrose; 4,6,6'-trichlorosucrose; 4,1',4',6'-tetrachlorogalactotagatose; 4,1',6'-trichlorogalactosucrose-6-acetate; and 4,6,1',6'-tetrachlorogalactosucrose.

Production of 4,1',6'-trichloro-4,1',6'-trideoxygalactosucrose and halogenated sugar derivatives, generically speaking, involves the substitution of halogen atoms in the sucrose molecule in one of its eight hydroxyl positions. The particular selection of positions determines the precise steps of the synthetic route. *See*, *e.g*., U.S. Pat. No. 4,380,476. The synthetic route may involve the preparation of an intermediate sucrose derivative having the required positions available for halogenation while the other positions are blocked. As an example of one synthetic route, one route for the production of 4,1',6'-trichloro-4,1',6-trideoxygalactosucrose (a tri-chlorinated sucrose) proposed in the literature involves the formation of the 6,1',6'-tritrityl derivative of sucrose, followed by peracetylation of the molecule and then detritylation with migration of the 4-acetyl radical to the 6-position, to give 2,3,6,3',4'-penta-O-acetylsucrose which has the correct hydroxy groups unprotected. Fairclough et al., 40 Carbohydrate Research 285-98 (1975). Subsequent reaction with a chlorinating agent provides the 4,1',6'-trichlorogalactosucrose penta-acetate which in turn yields sucralose on elimination of the acetyl groups. The chlorination proceeds with inversion of configuration. The 1' and 6'-positions freely rotate, but the 4-position cannot and the glucose ring is thus inverted at the 4-position into a galactose ring so that the product is a galactosucrose.

Halogenated sugar derivatives may be represented by the following generic formulae (I-III): 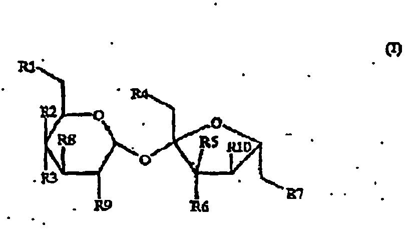 wherein
R1 represents a hydroxy group, an acyl group, or a halogen atom;
R2 and R3 respectively represent:
   (a) a hydrogen atom and a hydroxy group;
   (b) a hydrogen and a halogen atom;
   (c) a halogen atom and a hydrogen atom; or
   (d) a hydroxy group and a hydrogen atom;
R4, R5, R6, R7, R8, R9 and R10 represent a hydrogen atom, a hydroxy group, or a halogen atom; and wherein

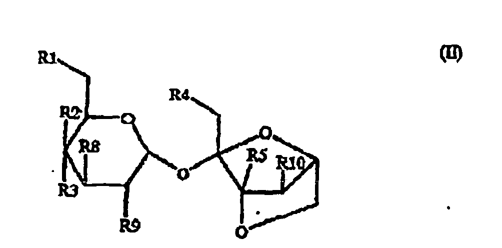

R1 represents a hydroxy group, an acyl group, or a halogen atom;
R2 and R3 respectively represent:
   (a) a hydrogen atom and a hydroxy group;
   (b) a hydrogen and a halogen atom;
   (c) a halogen atom and a hydrogen atom; or
   (d) a hydroxy group and a hydrogen atom;
R4, R5, R8, R9 and R10 represent a hydrogen atom, a hydroxy group, or a halogen atom; and
   wherein

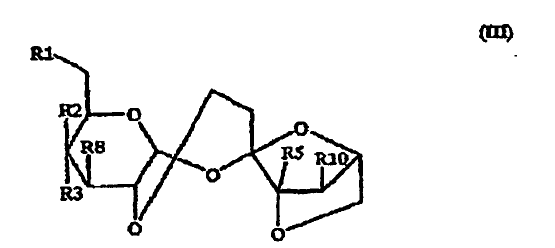

R1 represents a hydroxy group, an acyl group, or a halogen atom;
R2 and R3 respectively represent:
   (a) a hydrogen atom and a hydroxy group;
   (b) a hydrogen and a halogen atom;
   (c) a halogen atom and a hydrogen atom; or
   (d) a hydroxy group and a hydrogen atom; and
R5, R8, and R10 represent a hydrogen atom, a hydroxy group, or a halogen atom.

Hydroxy groups of a sugar such as, for example, sucrose may be blocked/deblocked in the course of a halogenation procédure. Halogens sultable for use in the context of the present invention include bromine, chlorine, fluorine, and lodine. One skilled in the art may readily fill the various positions with the same halogen or with any combination or permutation of different halogens by methods known to those skilled in the art.

Following synthesis, at least one helogenated sugar derivative is supplied with 4,1'6'-trichloro-4,1',6'-trideoxygalactosucrose. In one embodiment, the present inventions include compositions wherein halogenated sugar derivatives make up from about 0.001% to about 2.0% of the composition by weight and wherein 4,1',6'-trichloro-4,1'6'-trideoxygalactosucrose makes up from about 98.0% to about 99.999% of the composition by weight. In another embodiment, the present inventions include compositions wherein halogenated sugar derivatives make up from about 0.05% to about 2.0% of the composition by weight and wherein 4,1',6'-trichloro-4,1',6'-trideoxygalactosucrose makes up from about 98.0% to about 99.95% of the composition by weight. In yet another embodiment, the present inventions include compositions wherein halogenated sugar derivatives make up from about 0.1% to about 2.0% of the composition by weight and wherein 4,1',6'-trichloro-4,1',6'-trideoxygalactosucrose makes up from about 98.0% to about 99.9% of the composition by weight. In one embodiment, the present inventions include compositions comprising 4,1',6'-trichloro-4,1',6'-trideoxygalactosucrose and at least one halogenated sugar derivative wherein the weight ratio of at least one halogenated sugar derivative to the 4,1',6'-trichloro-4,1',6'-trideoxygalactosucrose is from about 1:99,999 to about 1:50. In another embodiment, the present inventions include compositions comprising 4,1'6'-trichloro-4,1'6'-trideoxygalactosucrose and at least one halogenated sugar derivative wherein the weight ratio of at least one halogenated sugar derivative to the 4,1',6'-trichloro-4,1',6'-trideoxygalactosucrose is from about 1:1,999 to about 1:50. In one embodiment, the present inventions include compositions comprising 4,1',6'-trichloro-4,1'6'-trideoxygalactosucrose and at least one halogenated sugar derivative wherein the weight ratio of at least one halogenated sugar derivative to the 4,1',6'-trichloro-4,1'6'-trideoxygalactosucrose is from about 1:999 to about 1:50. In an embodiment of the present invention, the compositions may comprise preservatives, binders, and/or carriers.

Surprisingly, this supplying of 4,1',6'-trichloro-4,1',6'-trideoxygalactosucrose with at least one halogenated sugar derivative leads to an alteration of the sweetness delivery profile of 4,1',6'-trichloro-4,1',6'-trideoxygalactosucrose. For example, such a combination may alter the manner in which 4,1',6'-trichloro-4,1',6'-trideoxygalactosucrose typically interacts with individual taste buds or groups of taste buds. In addition, such a combination may alter the manner in which 4,1',6'-trichloro-4,1',6'-trideoxygalactosucrose interacts with the lips, nose, cheeks, teeth, hard palate, soft palate, uvula, palatoglossal arches, palatopharyngeal arches, tongue, or any other component of the oral cavity known in the art. See, e.g., Elaine N. Marieb, Human Anatomy And Physiology 774-84 (2d ed. 1992). The alteration may include one or more of a change in sweetness acceptability, sweetness onset, sweetness intensity, aftertaste intensity, bitterness, sweetness duration, pleasantness of aftertaste, overall liking, and quality of taste as "artificial" or "natural," and others known to those skilled in the art.

Compositions of the present invention may be combined with combination sweeteners, consumer products, and beverages. In one embodiment, in the case of consumer products, such as baking mixes, one may add such a composition to a level of about 100 ppm to about 800 ppm. Alternately, in the case of consumer products, such as baking mixes, one may add such a composition to a level of about 200 ppm to about 600 ppm. Further, in the case of consumer products, such as baking mixes, one may add such a composition to a level of about 300 ppm to about 500 ppm. In addition, in the case of consumer products, such as baking mixes, one may add such a composition to a level of about 450 ppm. In one embodiment, in the case of beverages, such as water, one may add such a composition to a level of about 10 ppm to about 500 ppm. Alternately, in the case of beverages, such as water, one may add such a composition to a level of about 50 ppm to about 200 ppm. Further, in the case of beverages, such as water, one may add such a composition to a level of about 7S ppm to about 150 ppm. In addition, in the case of beverages such as water, one may add such a composition to a level of about 100 ppm.

### EXAMPLES

Without further elaboration, it is believed that one skilled in the art, using the preceding description, can utilize the present invention to the fullest extent. The following examples are illustrative only, and not limiting of the remainder of the disclosure in any way whatsoever.

In each of the following Examples, unless otherwise indicated, at least 100 panelists are preferably used to evaluate the samples, although fewer may be used. Two statistical tests are preferably used to determine if the addition of a halogenated sugar derivative significantly alters the various taste perceptions being studied. The first test, analysis of variance ("ANOVA"), was used here to determine whether two data sets (i.e., with and without a halogenated sugar derivative) differ at the 95% confidence level, once any variance between individual tasters is taken into account. The second, Tukey's HSD (Honestly Significant Difference) test, was also used here to determine whether data sets differ at the 95% confidence level. More specifically, Tukey's HSD test takes into account the mean square errors which have been determined by the ANOVA test. Both the ANOVA and Tukey's HSD tests are routinely used in the art.

### Example 1

A powdered composition comprising 4,1',6'-triChloro-4,1',6'-trideoxygalactosucrose and other halogenated sugar derivatives is obtained by a number of previously disclosed processes for synthesizing and purifying halogenated sugar derivatives. A test composition comprising about 98% to about 99.999% 4,1',6'-trichloro-4,1',6'-trideoxygalactosucrose by weight and about 0.001% to about 2% by weight of one or more halogenated sugar derivatives such as: 4,6'-dichlorogalactosucrose; 4,1'-dichlorogalactosucrose; 1',6'-dichlorosucrose; 3',6'-anhydro-4,1-dichlorogalactosucrose; 4,1',6'-trichlorogalactosucrose-6-acetate; and 6,1',6'-trichlorosucrose is obtained.

Three batches of aqueous solutions are prepared. Dissolved in the first batch (B₁) is essentially pure sucralose at a level of 100 ppm. Dissolved in the second batch (B₂) is the aforementioned test composition at a level of 100 ppm. Dissolved in the third batch (B₃) is an equally sweet amount of sucrose (table sugar); this equally sweet amount is determined by trained evaluators. 15 ml aliquots of the batches are prepared and appropriately labeled. Three aliquots--one from each batch--are presented to 100 panelists selected from the general population.

Each panelist takes 10 ml of the aliquot comprising sucrose into her mouth. After 10 seconds, the panelist swallows the sample. The panelist receives a plain cracker and a water rinse. The panelist rests for three minutes. Each panelist takes 10 ml of one of the other aliquots into his mouth. After 10 seconds, the panelist swallows the sample. The panelist receives a plain cracker and a water rinse. The panelist rests for three minutes. Each panelist takes 10 ml of the remaining aliquot into his mouth. After 10 seconds, the panelist swallows the sample. The panelist receives a plain cracker and a water rinse. The samples are randomly presented to panelists in order to avoid bias. Panelists are asked to record which of the final two samples tastes most like the first sample and record how confident they are of the result.

Statistical analyses of the results show that the aliquots from B₂ exhibit taste more similar to the taste of sucrose than do the aliquots from B₁. To a statistically significant degree (p less than or equal to 0.05), the data indicate that the presence of halogenated sugar derivatives in compositions comprising sucralose enhance the ability of sucralose to mimic table sugar.

### Example 2

A panel of six trained evaluators determine equally sweet levels of: 4,1',6'-trichloro-4,1',6'-trideoxygalactosucrose; 4,1',6'-trichloro-4,1',6'-trideoxygalactosucrose supplied with a halogenated sugar derivative, such as 4,6'-dichlorogalactosucrose, 4,1'-dichlorogalactosucrose, 1',6'-dichlorosucrose, 3',6'-anhydro-4,1-dichlorogalactosucrose, 4,1',6'-trichlorogalactosucrose-6-acetate, and 6,1',6'-trichlorosucrose; aspartame; and aspartame supplied with a halogenated sugar derivative such as 4,6'-dichlorogalactosucrose, 4,1'-dichlorogalactosucrose, 1',6'-dichlorosucrose, 3',6'-anhydro-4,1-dichlorogalactosucrose, 4,1',6'-trichlorogalactosucrose-6-acetate, and 6,1',6'-trichlorosucrose, all dissolved in non-carbonated bottled water.

For sweetness time-intensity studies, room temperature equally sweet solutions are presented to 12 trained panelists. The panelists are screened for general sensory acuity and trained in general methods for sweetener assessment as well as time-intensity methods. Training sessions are carried out so that all panelists are conversant with the method as well as the computerized data entry system.

For each evaluation, each panelist takes 10 ml of one of the solutions in his mouth and activates the computer timing system. Using a computer mouse, the panelist rates the sweetness intensity on a line scale (ranging from "none" to "extreme") while slowly moving the sample around in his mouth. After 10 seconds, the panelist swallows the sample and continues to rate for sweetness. Computer timing stops after a total of 180 seconds.

In each session, panelists receive two coded samples, with at least 10 minutes rest and plain cracker and water rinse between samples. Panelists rest for at least 30 minutes between sessions. No more than 3 sessions are allowed on one day. All samples are designated with a random 3-digit code and sample presentation order are randomized and balanced across panelists.

The data collected by the computer are read every second, for a total of 180 data points per tasting. Mean panelist response is calculated for each time data point. Analysis of variance is carried out to determine the differences between samples. The data indicate that a halogenated sugar derivative alters the sweetness delivery profile of both 4,1',6'-trichloro-4,1',6'-trideoxygalactosucrose and aspartame. To a statistically significant degree (p less than or equal to 0.05), halogenated sugar derivative-containing samples demonstrate differences from samples containing no halogenated sugar derivative with respect to one or more of the following attributes: first time that a maximum intensity is recorded, last time that a non-zero intensity is recorded, and last time that an intensity greater than 50% of the maximum intensity is recorded. These halogenated sugar derivative-containing samples demonstrate the utility of compositions and methods of the present invention.

### Example 3

This example illustrates the uniqueness of a sucralose-sweetened solution with a halogenated sugar derivative such as: 4,6'-dichlorogalactosucrose; 4,1'-dichlorogalactosucrose; 1',6'-dichlorosucrose; 3,6'-anhydro-4,1-dichlorogalactosucrose; 4,1',6'-trichlorogalactosucrose-6-acetate; and 6,1',6'-trichlorosucrose in accordance with the present invention. Two solutions are prepared. One solution has dissolved into it 39.52 g maltodextrin, 0.48 g sucralose, and 3,500 g water; the other solution has dissolved into it 39.52 g maltodextrin, 0.4752 g sucralose, 0.0048 g halogenated sugar derivative, and 3,500 g water. Maltodextrin present in the formulations is a carrier for sucralose and is controlled for by its use in both control and halogenated sugar derivative-containing samples.

Panelists receive a 20 ml serving of each solution. Panelists are asked to rate the compositions with respect to sweetness acceptability (rated on a five-point scale; 0 = poor, 3 = good, 5 = excellent), sweetness onset (rated on a seven point scale; 0 = dislike, 4 = just right, 7 = excellent), sweetness intensity (rated on a seven point scale; 0 = dislike, 4 = just right, 7 = excellent), aftertaste intensity (rated on a seven point scale; 0 = dislike, 4 = just right, 7 = excellent), bitterness (rated on a seven point scale; 0 = dislike, 4 = just right, 7 = excellent), sweetness duration (rated on a seven point scale; 0 = dislike, 4 = just right, 7 = excellent), pleasantness of aftertaste (rated on a seven point scale; 0 = dislike, 4 = just right, 7 = excellent), overall liking (rated on a nine point scale; 0 = dislike, 5 = just right, 9 = like extremely), and quality of taste as "artificial" or "natural" (rated on a 100 point unstructured scale; 0 = artificial, 100 = natural). The rates are averaged. Data are analyzed using Analysis of Variance and Tukey's HSD tests.

To a statistically significant degree (p less than or equal to 0.05), halogenated sugar derivative-containing samples demonstrate differences from samples containing no halogenated sugar derivative with respect to one or more of the following attributes: sweetness acceptability, sweetness onset, sweetness intensity, aftertaste intensity, bitterness, sweetness duration, pleasantness of aftertaste, overall liking, and quality of taste as "artificial" or "natural." These halogenated sugar derivative-containing samples demonstrate the utility of compositions and methods of the present invention.

### Example 4

Baked Apple Bar with SPLENDA® Brand Sweetener and a Halogenated Sugar Derivative Recipe Using SPLENDA® Brand Sweetener: 2 cups all purpose flour; 2 teaspoons baking powder; 1 teaspoon baking soda; 2 teaspoons ground cinnamon; 1 cup reduced-calorie margarine; 1/2 cup egg substitute; 2 teaspoons vanilla extract; 1/2 cup unsweetened applesauce; 2 cups peeled and grated apples; 50 g SPLENDA® Brand Sweetener (sucralose) crystalline powder.

Recipe Using SPLENDA® Brand Sweetener and a Halogenated Sugar Derivative: 2 cups all purpose flour; 2 teaspoons baking powder; 1 teaspoon baking soda; 2 teaspoons ground cinnamon; 1 cup reduced-calorie margarine; 1/2 cup egg substitute; 2 teaspoons vanilla extract; 1/2 cup unsweetened applesauce; 2 cups peeled and grated apples; 50 g SPLENDA® Brand Sweetener (sucralose) crystalline powder; and 0.006024 g of a halogenated sugar derivative such as: 4,6'-dichlorogalactosucrose; 4,1'-dichlorogalactosucrose; 1',6'-dichlorosucrose; 3',6'-anhydro-4,1-dichlorogalactosucrose; 4,1',6'-trichlorogalactosucrose-6-acetate; and 6,1',6'-trichlorosucrose.

The apple bars are prepared according to the following procedures:
1) Preheat oven to 350°F. Spray two 8x8x2" metal baking pans with vegetable cooking spray. In a small bowl, stir together flour, baking powder, baking soda, and cinnamon. Set aside.
2) In a large mixing bowl with mixer at high speed, beat margarine, about 1 minute. Add egg substitute and vanilla and blend at high speed for 30 seconds.
3) Add SPLENDA® Brand Sweetener (and halogenated sugar derivative when applicable), and beat at medium speed until smooth (∼90 seconds). Add apple sauce, grated apple, and flour mixture, and beat at low speed until mixed (∼45 seconds).
4) Spread batter in pan and bake for 40 minutes, or until a wooden pick inserted in the center comes out clean. Cool to room temperature and cut into bars.

The apple bars containing SPLENDA® Brand Sweetener are evaluated in comparison to the samples containing SPLENDA® with a halogenated sugar derivative. Panelists receive a standard-size serving of each bar. Panelists are asked to rate the compositions with respect to sweetness acceptability (rated on a five-point scale; 0 = poor, 3 = good, 5 = excellent), sweetness onset (rated on a seven point scale; 0 = dislike, 4 = just right, 7 = excellent), sweetness intensity (rated on a seven point scale; 0 = dislike, 4 = just right, 7 = excellent), aftertaste intensity (rated on a seven point scale; 0 = dislike, 4 = just right, 7 = excellent), bitterness (rated on a seven point scale; 0 = dislike, 4 = just right, 7 = excellent), sweetness duration (rated on a seven point scale; 0 = dislike, 4 = just right, 7 = excellent), pleasantness of aftertaste (rated on a seven point scale; 0 = dislike, 4 = just right, 7 = excellent), overall liking (rated on a nine point scale; 0 = dislike, 5 = just right, 9 = like extremely), and quality of taste as "artificial" or "natural" (rated on a 100 point unstructured scale; 0 = artificial, 100 = natural). The rates are averaged. Data are analyzed using ANOVA and Tukey's HSD tests.

To a statistically significant degree (p less than or equal to 0.05), the halogenated sugar derivative-containing samples demonstrate differences from the sample containing no halogenated sugar derivative with respect to one or more of the following attributes: sweetness acceptability, sweetness onset, sweetness intensity, aftertaste intensity, bitterness, sweetness duration, pleasantness of aftertaste, overall liking, and quality of taste as "artificial" or "natural." Such samples demonstrate the utility of the methods and compositions of the present invention.

### Example 5

### Cola Sweetened with Sucralose

Product Formula (Syrup Batch Formula): water (3074.6 g); cola concentrate (39.0 g); SPLENDA® Brand Sweetener (sucralose) liquid concentrate (16.6 g); phosphoric acid, 75% (10.8 g); potassium benzoate (5.0 g); citric add, anhydrous (2.0 g); caffeine, anhydrous (2.0 g).

### Preparation Procedure

(i) syrup: Add ingredients to water in the following order: potassium benzoate, SPLENDA® Brand Sweetener (sucralose) liquid concentrate, phosphoric acid, citric acid, caffeine and cola concentrate. Mix thoroughly between additions.
(ii) finished beverage: Mix syrup and carbonated water (one part syrup to five parts carbonated water) and bottle (CO₂ level of finished beverage = 3.6 volumes). The syrup batch makes 5 gallons of finished beverage.

Sensory research was conducted to determine the effect of a halogenated sugar derivative such as: 4,6'-dichlorogalactosucrose; 4,1'-dichlorogalactosucrose; 1',6'-dichlornsucrose; 3',6'-anhydro-4,1-dichlorogalactosucrose; 4,1',6'-richlorogalactosucrose-6-acetate; and 6,1',6'-trichlorosucrose on sucralose-sweetened cola. Three levels of halogenated sugar derivative were tested: 1 ppm, 2 ppm, and 5 ppm. Three groups of samples are produced, each with one of the alternate levels of halogenated sugar derivative.

Panelists are seated in individual, partitioned sensory booths to minimize their interaction with each other. Samples are presented one at a time. Each sample is evaluated before the next sample is tasted. Sample presentation order is randomized. Panelists consume two ounces of each refrigerated cola sample. After completing the evaluation of each sample, panelists are Instructed to rinse their mouths thoroughly with bites of cracker and bottled water.

Panelists receive a standard-size serving of each sample. Panelists are asked to rate the samples with respect to overall liking (rated on a nine point scale; 1 = dislike, 9 = like), sweetness amount (rated on a seven point scale; 1 = lack, 7 = abundance), sweetness quality (rated on a five point scale; 1 = poor, 5 = excellent), rate of sweetness onset (rated on a seven point scale; 1 = slow, 7 = rapid), cola flavor intensity (rated on a one hundred point scale; 0 = none, 100 = extreme), liking for cola flavor (rated on a nine point scale; 1 = dislike, 9 = like), bitterness intensity (rated on a five point scale; 1 = none, 5 = extreme), diet/regular taste (rated on a one hundred point scale; 0 = diet, 100 = regular), duration of sweet aftertaste (rated on a nine point scale; 1 = short, 9 = long), duration of cola flavor aftertaste (rated on a nine point scale; 1 = short, 9 = long), duration of other aftertaste (rated on a nine point scale; 1 = short, 9 = long), and pleasantness of aftertaste (rated on a seven point scale; 1 = unpleasant, 7 = pleasant). The rates are averaged. Data are analyzed using ANOVA and Tukey's HSD tests.

To a statistically significant degree (p less than or equal to 0.05), the halogenated sugar derivative-containing samples demonstrate differences from each other with respect to one or more of the following attributes: overall liking, sweetness amount, sweetness quality, rate of sweetness onset, cola flavor intensity, liking for cola flavor, bitterness Intensity, diet/regular taste, duration of sweet aftertaste, duration of cola flavor aftertaste, duration of other aftertaste, and pleasantness of aftertaste. These samples demonstrate the utility of compositions and methods of the present invention.

Various modifications and variations of the described methods and systems of the invention will be apparent to those skilled in the art without departing from the scope and spirit of the invention. Although the invention has been described in connection with specific preferred embodiments, it should be understood that the invention as claimed should not be unduly limited to such specific embodiments. It is expressly intended, for example, that all ranges broadly recited in this document include within their scope all narrower ranges which fall within the broader ranges. Indeed, various modifications of the described modes for carrying out the invention which are apparent to those skilled in the art are intended to be within the scope of the following claims.

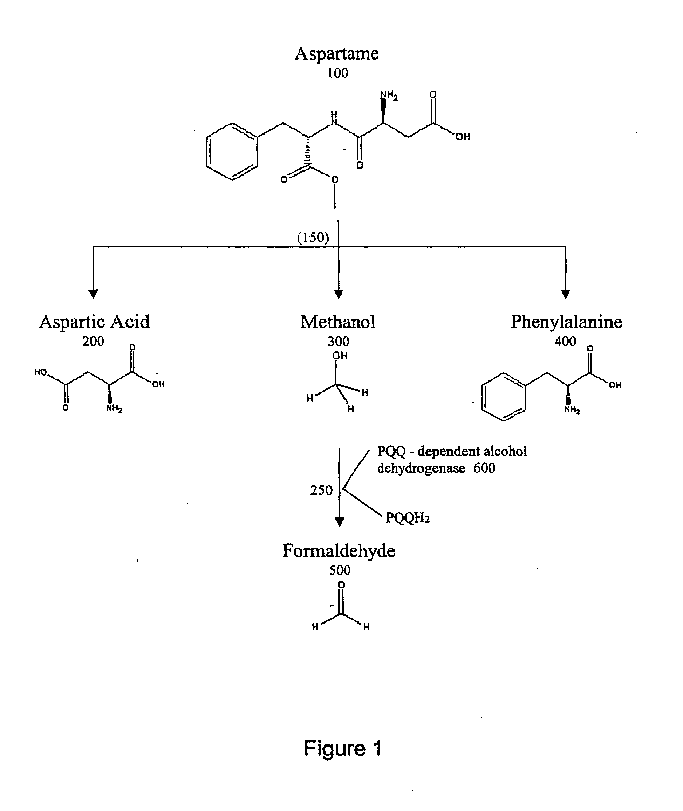

## Claims

1. A composition comprising 4, 1', 6'-trichloro-4, 1', 6'-trideoxygalactosucrose in an amount from 98.0% to 99.999% of said composition by weight, at least one halogenated sugar derivative of the following formula (I), and at least one halogenated sugar derivative selected from the group of the following formulae (II-III) : 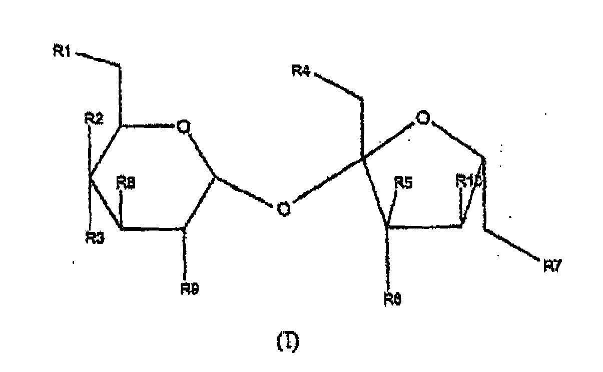 wherein
R1 represents a hydroxy group, an acyl group, or a halogen atom;
R2 and R3 respectively represent:
(a) a hydrogen atom and a hydroxy group;
(b) a hydrogen and a halogen atom ;
(c) a halogen atom and a hydrogen atom; or
(d) a hydroxy group and a hydrogen atom;
R4, R5, R6, R7, R8, R9 and R10 represent a hydrogen atom, a hydroxy group, or a halogen atom; and 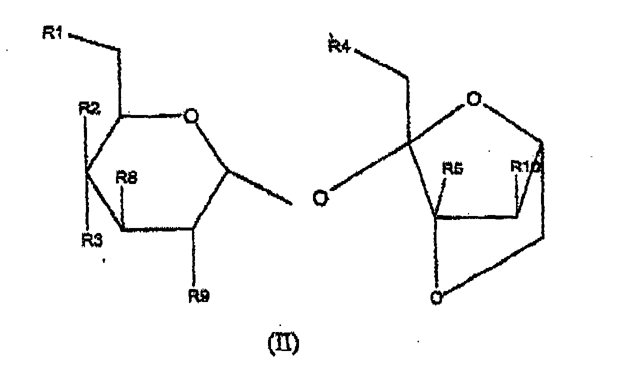
wherein
R1 represents a hydroxy group, an acyl group, or a halogen atom;
R2 and R3 respectively represent:
(a) a hydrogen atom and a hydroxy group;
(b) a hydrogen and a halogen atom;
(c) a halogen atom and a hydrogen atom; or
(d) a hydroxy group and a hydrogen atom;
R4, R5, R8, R9 and R10 represent a hydrogen atom, a hydroxy group, or a halogen atom; and 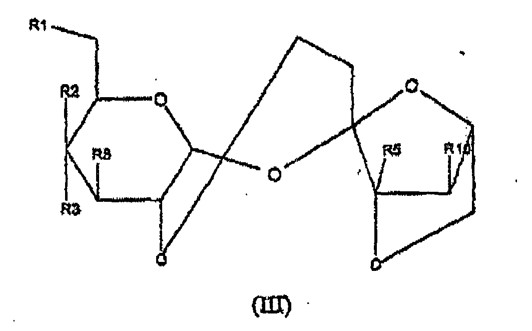
wherein
R1 represents a hydroxy group, an acyl group, or a halogen atom;
R2 and R3 respectively represent:
(a) a hydrogen atom and a hydroxy group;
(b) a hydrogen and a halogen atom;
(c) a halogen atom and a hydrogen atom; or
(d) a hydroxy group and a hydrogen atom; and
R5, R8, and R10 represent a hydrogen atom, a hydroxy group, or a halogen atom.

2. A consumer product comprising the composition of claim 1.

3. A combination sweetener comprising the composition of claim 1.

4. A beverage comprising the composition of claim 1.

5. A method for altering the sweetness delivery profile of 4, 1', 6'-trichloro-4,1',6'-trideoxygalactosucrose comprising the step of supplying a composition according to claim. 1.

## Patentansprüche

1. Zusammensetzung umfassend 4,1',6'-trichloro-4,1',6'-tridesoxygalactosaccharose in einer Menge von 98,0% bis 99,999 Gew.-% der Zusammensetzung, mindestens ein halogeniertes Zuckerderivat der folgenden Formel (I), und mindestens ein halogeniertes Zuckerderivat ausgewählt aus der Gruppe der folgenden Formel (II-III); 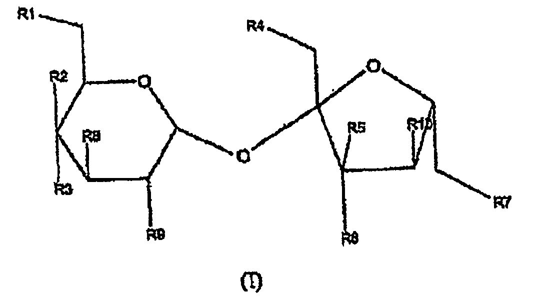 wobei
R1 eine Hydroxygruppe, eine Acylgruppe, oder ein Halogenatom darstellt;
R2 und R3 jeweils Folgendes darstellen:
(a) ein Wasserstoffatom und eine Hydroxygruppe;
(b) ein Wasserstoffatom und ein Halogenatom;
(c) ein Halogenatom und ein Wasserstoffatom; oder
(d) eine Hydroxygruppe und ein Wasserstoffatom;
R4, R5, R6, R7, R8, R9 und R10 ein Wasserstoffatom, eine Hydroxygruppe, oder ein Halogenatom darstellen; und 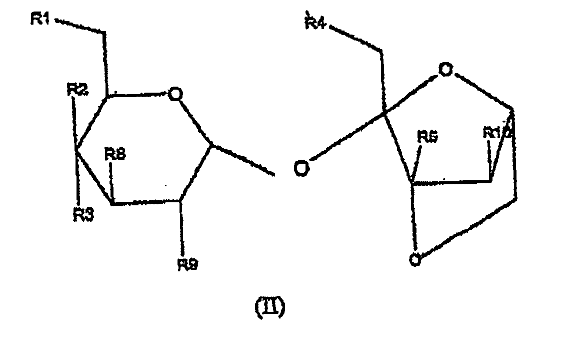
wobei
R1 eine Hydroxygruppe, eine Acylgruppe, oder ein Halogenatom darstellt;
R2 und R3 jeweils Folgendes darstellen:
(a) ein Wasserstoffatom und eine Hydroxygruppe;
(b) ein Wasserstoffatom und ein Halogenatom;
(c) ein Halogenatom und ein Wasserstoffatom; oder
(d) eine Hydroxygruppe und ein Wasserstoffatom;
R4, R5, R8, R9 und R10 ein Wasserstoffatom, eine Hydroxygruppe, oder ein Halogenatom darstellen; und 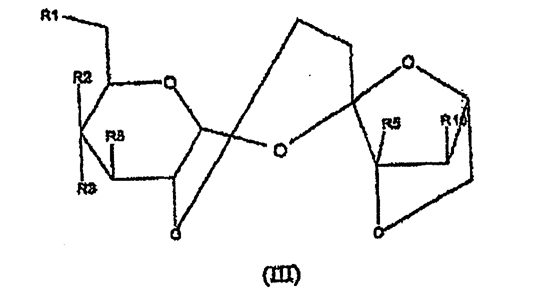
wobei
R1 eine Hydroxygruppe, eine Acylgruppe, oder ein Halogenatom darstellt;
R2 und R3 jeweils Folgendes darstellen:
(a) ein Wasserstoffatom und eine Hydroxygruppe;
(b) ein Wasserstoffatom und ein Halogenatom;
(c) ein Halogenatom und ein Wasserstoffatom; oder
(d) eine Hydroxygruppe und ein Wasserstoffatom; und
R5, R8 und R10 ein Wasserstoffatom, eine Hydroxygruppe, oder ein Halogenatom darstellen.

2. Konsumartikel umfassend die Zusammensetzung nach Anspruch 1.

3. Kombinationssüßungsmittel umfassend die Zusammensetzung nach Anspruch 1.

4. Getränk umfassend die Zusammensetzung nach Anspruch 1.

5. Verfahren zum Umwandeln des Süße-Zuführungsprofils von 4,1',6'-trichloro-4,1',6'-tridesoxygalactosaccharose umfassend den Schritt des Bereitstellens der Zusammensetzung nach Anspruch 1.

## Revendications

1. Composition comprenant 4, 1', 6'-trichloro-4, 1', 6'-tridésoxygalactosaccharose dans une quantité de 98,0 % à 99,999 % en poids de ladite composition, au moins un sucre halogéné dérivé de la formule (I) suivante, et au moins un sucre halogéné dérivé sélectionné à partir du groupe de la formule (II-III) suivante : 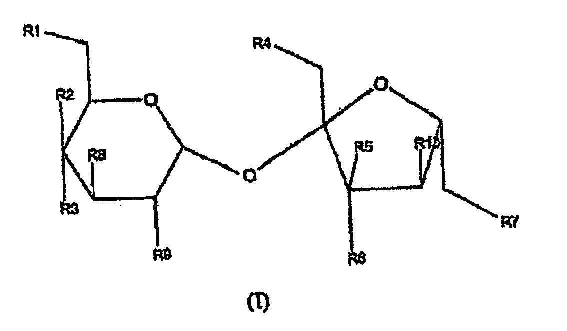 où
R1 représente un groupe hydroxy, un groupe acyle, ou un atome halogène ;
R2 et R3 représentent respectivement :
(a) un atome hydrogène et un groupe hydroxy ;
(b) un atome hydrogène et un atome halogène ;
(c) un atome halogène et un atome hydrogène ; ou
(d) un groupe hydroxy et un atome hydrogène ;
R4, R5, R6, R7, R8, R9 et R10 représentent un atome hydrogène, un groupe hydroxy, ou un atome halogène ; et 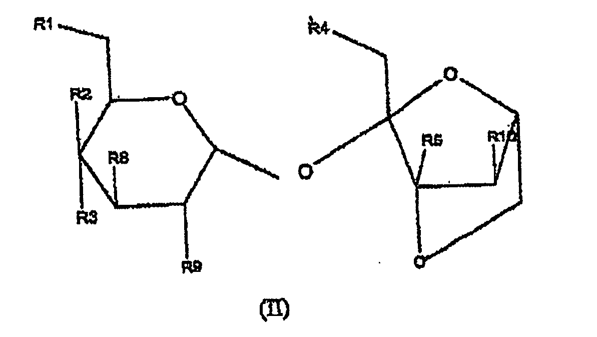
où
R1 représente un groupe hydroxy, un groupe acyle, ou un atome halogène ;
R2 et R3 représentent respectivement :
(a) un atome hydrogène et un groupe hydroxy ;
(b) un atome hydrogène et un atome halogène ;
(c) un atome halogène et un atome hydrogène ; ou
(d) un groupe hydroxy et un atome hydrogène ; et
R4, R5, R8, R9 et R10 représentent un atome hydrogène, un groupe hydroxy, ou un atome halogène ; et 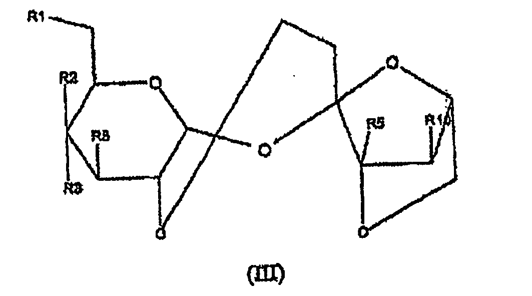
où
R1 représente un groupe hydroxy, un groupe acyle, ou un atome halogène ;
R2 et R3 représentent respectivement :
(a) un atome hydrogène et un groupe hydroxy ;
(b) un atome hydrogène et un atome halogène ;
(c) un atome halogène et un atome hydrogène ; ou
(d) un groupe hydroxy et un atome hydrogène ; et
R5, R8, et R10 représentent un atome hydrogène, un groupe hydroxy, ou un atome halogène.

2. Produit de consommation comprenant la composition de la revendication 1.

3. Combinaison édulcorante comprenant la composition de la revendication 1.

4. Boisson comprenant la composition de la revendication 1.

5. Procédé en vue d'altérer le profil de délivrance de teneur en sucre de 4, 1', 6'-trichloro-4, 1', 6'-tridésoxygalactosaccharose comprenant l'étape de fourniture d'une composition selon la revendication 1.
